# EUROPEAN PATENT SPECIFICATION

(11) **EP 0 693 288 B1**
(45) Date of publication and mention of the grant of the patent: **13.10.1999**
(21) Application number: 95111117.8
(22) Date of filing: 09.04.1991
(51) Int. Cl.: A61K 49/00

(54) **Polymers as contrast media for magnetic resonance**
Polymere als Kontrastmittel bei magnetischer Resonanz
Polymères commes substances de contraste pour résonance magnétique

(30) Priority: 10.04.1990 US 507125
(43) Date of publication of application: 24.01.1996
(62) Divisional of application: 91908011.9
(73) Proprietor: IMARX PHARMACEUTICAL CORP., Tucson, AZ 85719 (US)
(72) Inventor: Unger, Evan C, Tucson, AZ 85749 (US)
(74) Representative: Stebbing, Peter John Hunter

(56) References cited:
- EP-A- 0 118 281
- EP-A- 0 184 899
- EP-A- 0 228 692
- EP-A- 0 284 549
- EP-A- 0 409 351
- WO-A-85/04330
- WO-A-89/11874
- WO-A-90/01953
- US-A- 4 719 098
- US-A- 4 729 892
- US-A- 4 822 594
- US-A- 4 849 210
- US-A- 4 871 716
- US-A- 4 933 441

## Description

This invention relates to the field of magnetic resonance imaging, more specifically to the use of polymers optionally in combination with contrast agents and/or gases as contrast media for magnetic resonance imaging.

There are a variety of imaging techniques that have been used to diagnose disease in humans. One of the first imaging techniques employed was X-rays. In X-rays, the images produced of the patients' body reflect the different densities of body structures. To improve the diagnostic utility of this imaging technique, contrast agents are employed to increase the density between various structures, such as between the gastrointestinal tract and its surrounding tissues. Barium and iodinated contrast media, for example, are used extensively for X-ray gastrointestinal studies to visualize the esophagus, stomach, intestines and rectum. Likewise, these contrast agents are used for X-ray computed tomographic studies to improve visualization of the gastrointestinal tract and to provide, for example, a contrast between the tract and the structures adjacent to it, such as the vessels or lymph nodes. Such gastrointestinal contrast agents permit one to increase the density inside the esophagus, stomach, intestines and rectum, and allow differentiation of the gastrointestinal system from surrounding structures.

Magnetic resonance imaging (MRI) is a relatively new imaging technique which, unlike X-rays, does not utilize ionizing radiation. Like computed tomography, MRI can make cross- sectional images of the body, however MRI has the additional advantage of being able to make images in any scan plane (i.e., axial, coronal, sagittal or orthogonal). Unfortunately, the full utility of MRI as a diagnostic modality for the body, particularly in the abdominal and pelvic region, is hampered by the lack of an effective gastrointestinal contrast agent. Without such an agent, it is often difficult using MRI to differentiate the intestines from, for example, adjacent soft tissues and lymph nodes. If better contrast agents were available, the overall usefulness of MRI as an imaging agent would improve, and the diagnostic accuracy of this modality in the gastrointestinal region would be greatly enhanced.

MRI employs a magnetic field, radiofrequency energy and magnetic field gradients to make images of the body. The contrast or signal intensity differences between tissues mainly reflect the T1 and T2 relaxation values and the proton density (effectively, the free water content) of the tissues. In changing the signal intensity in a region of a patient by the use of a contrast medium, several possible approaches are available. For example, a contrast medium could be designed to change either the T1, the T2 or the proton density.

A paramagnetic contrast agent such as Gd-DTPA causes longitudinal relaxation to shorten T1. This increases the signal intensity on T1-weighted images. A superparamagnetic contrast agent such as ferrites works predominantly on transverse relaxation causing a shortening of T2 and decreasing signal intensity on T2-weighted images. A contrast agent could also work by altering the proton density, specifically by decreasing the amount of free water available that gives rise to the signal intensity.

Agents that increase the signal intensity from the lumen compared to the native contents are termed positive contrast agents. A number of these have been examined as contrast agents for MRI. These include fats and oils (Newhouse et al., Radiology, 142(P):246 (1982)), which increase signal as a result of their short T1, long T2 and high intrinsic proton density, as well as various paramagnetic agents that increase signal by decreasing the T1 of water protons. Examples of such paramagnetic agents include Gd-DTPA (Kornmesser et al., Magn. Reson. Imaging, 6:124 (1988), and Laniado et al., AJR, 150:817 (1988)), Gd-DOTA (Hahn et al. Magn. Reson. Imaging, 6:78 (1988)), Gd-oxalate (Runge, V.M. et al., Radiology, 147:789 (1983)), Cr-EDTA (Runge, V.M. et al., Physiol. Chem. Phys. Med. NMR, 16:113 (1984)), Cr-Trisacetylacetonate (Clanton et al., Radiology, 149:238 (1983)), ferric chloride (Young et al., CT, 5:543 (1981)), ferrous gluconate (Clanton et al., Radiology, 153:159 (1984)), ferric ammonium citrate and ferrous sulfate (Wesbey et al., Radiology, 149:175 (1983) and Tscholakoff et al., AJR, 148:703 (1987)) as well as iron complexes (Wesbey et al., Magn. Reson. Imaging, 3:57 (1985) and Williams et al., Radiology, 161:315 (1986)).

Alternatively, agents that decrease the signal intensity from the lumen are termed negative contrast agents. Examples include particulate iron oxides (Hahn et al., Radiology, 164:37 (1987), Widder et al., AJR, 149:839 (1987)) which decrease signal via T2 shortening, as well as gas-evolving materials (Weinreb et al., J. Comput. Assist. Tomogr., 8:835 (1984)) and perfluorocarbons (Mattrey et al., AJR, 148:1259 (1987)) which act through changes in the proton density. It should be recognized that all paramagnetic substances at sufficiently high concentrations can also result in a decrease in signal intensity via T2 shortening.

The existing MRI contrast agents all suffer from a number of limitations when employed as oral gastrointestinal agents. Positive contrast agents increase the image noise arising from intrinsic peristaltic motions and motions imposed via respiration or cardiovascular action. Positive contrast agents such as Gd-DTPA are subject to the further complication that the signal intensity depends upon the concentration of the agent as well as the pulse sequence used. Absorption of contrast agent from the gastrointestinal tract complicates interpretation of the images, particularly in the distal portion of the small intestine, unless sufficiently high concentrations of the paramagnetic species are used (Kornmesser et al., Magn. Reson. Imaging, 6:124 (1988)). Negative contrast agents by comparison are less sensitive to variation in pulse sequence and provide more consistent contrast. However at high concentrations, particulates such as ferrites can cause magnetic susceptibility artifacts which are particularly evident in the colon where the absorption of intestinal fluid occurs and the superparamagnetic material may be concentrated. Negative contrast agents typically exhibit superior contrast to fat, however on T1-weighted images, positive contrast agents exhibit superior contrast versus normal tissue. Since most pathological tissues exhibit longer T1 and T2 than normal tissue, they will appear dark on T1-weighted and bright on T2-weighted images. This would indicate that an ideal contrast agent should appear bright on T1-weighted images and dark on T2-weighted images. None of the currently available MRI contrast media for use with the gastrointestinal tract meet these dual criteria.

Toxicity is another problem with the existing contrast agents. With any drug there is some toxicity, the toxicity generally being dose related. With the ferrites there are often symptoms of nausea after oral administration, as well as flatulence and a transient rise in serum iron. The paramagnetic contrast agent Gd-DTPA is an organometallic complex of gadolinium coupled with the complexing agent diethylene triamine pentaacetic acid. Without coupling, the free gadolinium ion is highly toxic. The peculiarities of the gastrointestinal tract, wherein the stomach secretes acids and the intestines release alkalines, raise the possibility of decoupling and separation of the free gadolinium from the complex as a result of these changes in pH during gastrointestinal use. Certainly, minimizing the dose of either gastrointestinal contrast agent, whether paramagnetic or superparamagnetic, is important for minimizing any potential toxic effects.

New and/or better contrast agents useful in magnetic resonance imaging, particularly in the imaging of the gastrointestinal tract but also in the imaging of other regions of the body such as through the vasculature, are needed. The present invention is directed to this important end.

The present invention is directed to a contrast medium for magnetic resonance imaging, said contrast medium comprising an aqueous solution of at least one biocompatible non-cross-linked polymer and a biocompatible gas. Optionally, the contrast medium further comprises, in admixture, contrast agents, especially paramagnetic, superparamagnetic and/or proton density contrast agents. The polymer or polymer and contrast agent admixtures also comprise biocompatible gases, preferably gases such as air, oxygen, carbon dioxide, nitrogen, xenon, neon and/or argon.

The subject invention is applicable to a method of providing an image of an internal region of a patient, especially an image of the gastrointestinal region of the patient to which has been administered one or more of the aforementioned contrast agents, which comprises scanning the patient using magnetic resonance imaging to obtain visible images of the region.

Finally, the present invention is useful in a method for diagnosing the presence of diseased tissue in the patient.

Aspect of the invention will become more apparent from the following detailed description when taken in conjunction with the following drawings.
Fig. 1 is a diagrammatic view of a contrast medium in accordance with the present invention;
Fig. 2 is a graph of relaxation rate (1/sec) versus percent polyethylene glycol (w/w), wherein the polyethylene glycol polymer is in aqueous solution both with and without a Gd-DTPA contrast agent;
Fig. 3 is a graph of relaxation rate (1/sec) versus percent dextrose (w/w), wherein the dextrose polymer is in aqueous solution both with and without a Gd-DTPA contrast agent;
Fig. 4 is a graph of 1/T2 (1/sec) versus ferrite concentration (micromolar), wherein the ferrite contrast agent is in aqueous solution with and without a cellulose polymer (and wherein the cellulose polymer is present with and without carbon dioxide gas).

Any of the wide variety of biocompatible polymers known in the art may be employed in the medium or methods of the subject invention. As will be readily apparent to those skilled in the art, there are numerous types of such polymers available. Preferably, the polymer chosen is one which has a relatively high water binding capacity. Also preferably, the polymer has limited ability for ion complexation. Where imaging of the gastrointestinal region is desired, preferably the polymer chosen is one which is not substantially absorbed from or degraded within the gastrointestinal region. The polymers useful in the present invention can be of either synthetic or natural origin. As used herein, the term polymer denotes a compound comprised of two or more repeating monomeric units, and preferably 10 or more repeating monomeric units. The polymers may be cross-linked, if desired. Preferably, however, the polymers are not cross-linked.

Exemplary synthetic polymers suitable for use in the present invention include polyethylenes (such as, for example, polyethylene glycol), polyoxyethylenes (such as, for example,polyoxyethylene glycol), polypropylenes (such as, for example, polypropylene glycol), pluronic acids and alcohols, polyvinyls (such as, for example, polyvinyl alcohol), and polyvinyl-pyrrolidone. Exemplary natural polymers suitable for use in the present invention include polysaccharides. Such polysaccharides include, for example, arabinans, fructans, fucans, galactans, galacturonans, glucans, mannans, xylans (such as, for example, inulin), levan, fucoidan, carrageenan, galactocarolose, pectic acid, amylose, pullulan, glycogen, amylopectin, cellulose, carboxylmethylcellulose, hydroxypropyl methylcellulose, dextran, pustulan, chitin, agarose, keratan, chondroitin, dermatan, hyaluronic acid and alginic acid, and various other homopolymers or heteropolymers such as those containing one or more of the following aldoses, ketoses, acids or amines: erythrose, threose, ribose, arabinose, xylose, lyxose, allose, altrose, glucose, mannose, gulose, idose, galactose, talose, erythrulose, ribulose, xylulose, psicose, fructose, sorbose, tagatose, glucuronic acid, gluconic acid, glucaric acid, galacturonic acid, mannuronic acid, glucosamine, galactosamine and neuraminic acid.

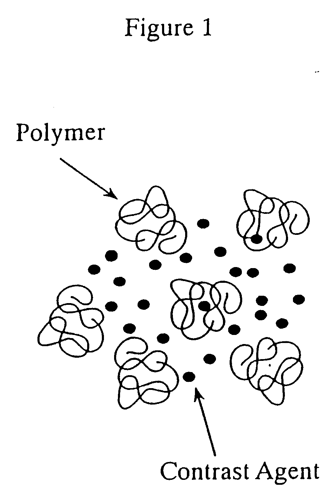

Polyethylene glycol (PEG), a polymer that exhibits a high water binding capacity, is particularly preferred for use in the subject invention. As a result of their high water binding capacity and concomitant decrease in the amount of free water in solution, PEG and similar polymers serve to alter the proton density in solution. Furthermore, PEG is used for the fractional precipitation of proteins from solution, which is believed to be due to in part to the excluded volume effects caused by this polymer whereby the protein is excluded from regions of the solution occupied by the polymer and is concentrated up in the water spaces, that is, the extrapolymer spaces, between the individual molecules of the polymer. This exclusion and concentration effect is illustrated diagrammatically in Figure 1, with the polymer being represented by the squiggly lines and the contrast agent being represented by the solid dots. Since PEG exhibits limited ability for ion complexation, it will also cause a small paramagnetic chelate such as Gd-DTPA to be concentrated such that the effective concentration and the relaxivity of the paramagnetic species will be higher in mixtures with the polymer than in the absence of the polymer. For these and other reasons, PEG, and related polymers, are particularly preferred polymers for the subject invention.

For reasons of diagnostic efficacy, other preferable polymers include polygalacturonic acid and dextran.

The polymers of the present invention may be employed with the biocompatible gas in an aqueous solution, as a contrast medium for magnetic resonance imaging. Alternatively, if desired, the polymer/gas admixture may be employed with conventional contrast agent. By admixture, it is meant that the contrast agent is simply added to the polymer-containing medium, and is not chemically bound to the polymer by a covalent linkage. Electrostatic interactions or hydrogen bonding may, however, exist between the polymer and contrast agents, and such associations are considered to be within the ambit of the term admixture.

Numerous contrast agents are well known to those skilled in the art and include, for example, paramagnetic, superparamagnetic and proton density contrast agents.

Exemplary paramagnetic contrast agents suitable for use in the subject invention include stable free radicals (such as, for example, stable nitroxides), as well as compounds comprising transition, lanthanide and actinide elements covalently or noncovalently bound to complexing agents or to proteinaceous macromolecules. Preferable elements include Gd(III), Mn(II), Cu(II), Cr(III), Fe (II) , Fe (III), Co(II), Er(II), Ni (II), Eu(III) and Dy(III). More preferably, the elements include Gd(III), Mn(II), Cu(II), Fe (II), Fe(III), Eu(III) and Dy(III), especially Gd(III). Preferable complexing agents include, for example, diethylenetriamine-pentaacetic acid (DTPA), ethylenediaminetetraacetic acid (EDTA), 1,4,7,10-tetraazacyclododecane-N,N',N',N'''-tetraacetic acid (DOTA), 1,4,4,7,10-tetraazacyclododecane-N,N',N''-triacetic acid (DO3A), 3,6,9-triaza-12-oxa-3,6,9-tricarboxymethylene-10-carboxy-13-phenyl-tridecanoic acid (B-19036), hydroxybenzylethylene-diamine diacetic acid (HBED), N,N'-bis(pyridoxyl-5-phosphate)ethylene diamine, N,N'-diacetate (DPDP), 1,4,7-triazacyclononane-N,N',N''-triacetic acid (NOTA), 1,4,8,11-tetraazacyclotetradecane-N,N'N'', N'''-tetraacetic acid (TETA) and kryptands (that is, macrocyclic complexes). More preferably, the complexing agents are DTPA, DOTA, DO3A and kryptands, most preferably DTPA. Preferable proteinaceous macromolecules include albumin, collagen, polyarginine, polylysine, polyhistidine, γ-globulin and ß-globulin. More preferably, the proteinaceous macromolecules comprise albumin, polyarginine, polylysine, and polyhistidine. Most preferably, the paramagnetic contrast agents employed in the present invention are Gd(III)-DTPA, Gd(III)-DOTA, Gd(III)-DO3A, or Gd(III)-kryptands, especially Gd(III)-DTPA.

Exemplary superparamagnetic contrast agents suitable for use in the subject invention include ferro- or ferrimagnetic compounds, such as pure iron, magnetic iron oxide (such as magnetite), γ-Fe₂O₃, manganese ferrite, cobalt ferrite and nickel ferrite.

Exemplary proton density contrast agents include perfluorocarbons.

The polymer or polymer and contrast agent admixtures of the subject invention are employed, in admixture with biocompatible gases. In the case of both negative and positive contrast agents, such gases serve to increase the efficacy of the contrast medium. The gas can be bubbled through the medium using conventional techniques. Any biocompatible gas is suitable for use in the present invention. Numerous such gases are well known to those skilled in the art. Exemplary biocompatible gases include, air, oxygen, carbon dioxide, nitrogen, xenon, neon and argon.

Wide variations in the amounts of the polymer. gas, and the contrast agent can be employed in the contrast medium of the invention. Preferably, however, the polymer is present in a concentration of at least about 1%, by weight, more preferably, between about 5% and about 50%, by weight, and generally most preferably at about 40%, by weight. Of course, as those skilled in the art would recognize, within these parameters the optimum polymer concentration will be influenced by the molecular weight of the polymer, its water binding capacity, as well as other characteristics of the particular polymer employed. Also, preferably, in the case of paramagnetic and proton density contrast agents, the contrast agent is present in a concentration of at least about 0.1 millimolar, more preferably between about 0.5 and about 2 millimolar, most preferably at about 1 millimolar. In the case of superparamagnetic agents, the concentration is preferably at least about 1 micromolar, more preferably between about 5 and about 50 micromolar, and most preferably is about 10 micromolar. The gas employed is preferably bubbled at at least about 20 psi through the solution for about one minute prior to administration, more preferably between about 30 or about 50 psi, most preferably about 40 psi.

The present invention is useful in imaging a patient generally, and/or in specifically diagnosing the presence of diseased tissue in a patient. The imaging process of the present invention may be carried out by administering a contrast medium of the invention to a patient, and then scanning the patient using magnetic resonance imaging to obtain visible images of an internal region of a patient and/or of any diseased tissue in that region. By region of a patient, it is meant the whole patient or a particular area or portion of the patient. The contrast medium is particularly useful in providing images of the gastrointestinal region, but can also be employed more broadly such as in imaging the vasculature or in other ways as will be readily apparent to those skilled in the art. The phrase gastrointestinal region or gastrointestinal tract, as used herein, includes the region of a patient defined by the esophagus, stomach, small and large intestines and rectum. The phrase vasculature, as used herein, denotes the blood vessels in the body or in an organ or part of the body. The patient can be any type of mammal, but most preferably is a human.

As one skilled in the art would recognize, administration may be carried out in various fashions, such as intravascularly, orally, rectally, etc., using a variety of dosage forms. When the region to be scanned is the gastrointestinal region, administration of the contrast medium of the invention is preferably carried out orally or rectally. The useful dosage to be administered and the particular mode of administration will vary depending upon the age, weight and the particular mammal and region thereof to be scanned, and the particular medium of the invention to be employed. Typically, dosage is initiated at lower levels and increased until the desired contrast enhancement is achieved. Various combinations of biocompatible polymers may be used to modify the relaxation behavior of the medium or to alter properties such as the viscosity, osmolarity or palatability (in the case of orally administered materials). In carrying out the method of the present invention, the contrast medium can be used alone, or in combination with other diagnostic, therapeutic or other agents. Such other agents include excipients such as flavoring or coloring materials. In addition, if desired, the contrast media of the invention may be encapsulated in liposomes or in other delivery vehicles. The polymer and gas and where applicable the contrast agent admixture may be sterilized by autoclaving prior to use, if desired.

The magnetic resonance imaging techniques which are employed are conventional and are described, for example, in D.M. Kean and M.A. Smith, Magnetic Resonance Imaging: Principles and Applications, (William and Wilkins, Baltimore 1986). Contemplated MRI techniques include, but are not limited to, nuclear magnetic resonance (NMR) and electronic spin resonance (ESR). The preferred imaging modality is NMR.

As will be apparent to those skilled in the art. the inventive admixture when employed in magnetic resonance imaging, may operate as a T1, T2 or proton density contrast medium, depending upon the type of polymer used, the molecular weight of the polymer, the concentration of the polymer, the type of contrast agent mixed with the polymer, the type of MRI modality chosen, and the details of the pulse sequence employed for MRI imaging, and all such mechanisms of operation are considered to be within the ambit of the present invention.

The media of the present invention have been shown to be extremely useful as contrast enhancement media in magnetic resonance imaging, particularly in imaging of the gastrointestinal region. By employing the inventive admixture accordance with the present invention, lower overall concentrations of the contrast agents may be used to achieve the same, or in many cases a better degree of, contrast enhancement results. This has benefits not only in terms of toxicity, by avoiding the use of large amounts of the potentially toxic contrast agents, but also in terms of cost, since less of the often more expensive conventional contrast agents are used. Furthermore, in the case of negative contrast agents based on superparamagnetic particles, magnetic susceptibility artifacts will be reduced through the ability to use a lower dose of the contrast agent. These and other advantages described herein of the present invention will be readily apparent to those skilled in the art, upon reading the present disclosure.

The present invention is further described in the following Examples. These Examples are not to be construed with as limiting the scope of the appended Claims.

### EXAMPLES

### Example 1

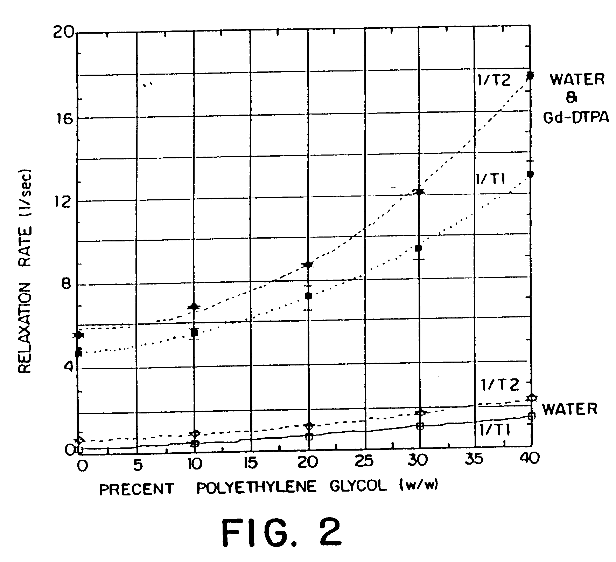

The polymer polyethylene glycol (PEG), having a molecular weight about 8000, was dissolved in water to various concentrations (w/w; by weight). To some of the aqueous PEG solutions was then added the contrast agent Gd-DTPA, such that the final concentration of Gd-DTPA was 1 mM. The relaxivity (1/T1 and 1/T2) of the PEG and the PEG and Gd-DTPA solutions was then tested in vitro using a Toshiba MR-50A 0.5 Tesla (T) whole body scanner. The results are shown in Table 1 and Figure 2. As the results indicate, in the presence of PEG the relaxivity of both water and Gd-DTPA is increased.

At best, it would be expected that the relaxation rates would be simply additive, i.e. the relaxation rate observed would be the sum of the relaxation rates of the individual components. However, an inspection of Table 1 and Figure 2 shows that the T1 relaxation rate of 40% (w/w) PEG 8000 in water was 1.35 ± 0.04 at 0.5 T and the relaxation rate for 1mM Gd-DTPA in water was measured to be 4.68 ± 0.09 at 0.5 T. If the rates were simply additive it would be expected that the observed relaxation rate for 1 mM Gd-DTPA in a 40% (w/w) PEG 8000 solution would be approximately 4.68 ± 1.35 = 6.03, at 0.5 T. The results in Table 1 and Figure 2, however, Surprisingly reveal that the relaxation rate for the PEG/water/Gd-DTPA mixture was in fact 12.81 ± 0.72 at 0.5 T. In sum, for both T1 and T2 relaxation rates, it has been observed that the relaxation rate of the polymer/Gd-DTPA admixture is greater than the sum of the relaxation rates of the PEG solution and the Gd-DTPA solution alone.

The foregoing result is in hindsight believed to arise as a consequence of the exclusion of the Gd-DTPA from the immediate environment of the PEG molecules so that the effective concentration of the Gd-DTPA is increased in the water not bound to the polymer. However, the present invention is not intended to be limited by any theory of operation.

**Table 1**

| Relaxivities at 0.5 T for PEG 8000/Water Mixtures In the Absence and Presence of 1mM Gd-DTPA | | |
|---|---|---|
| Sample | 1/T1(1/sec) | 1/T2 (1/sec) |
| water | 0.21 ± 0.04 | 0.65 ± 0.04 |
| 10% PEG/water | 0.41 ± 0.03 | 0.85 ± 0.03 |
| 20% PEG/water | 0.64 ± 0.02 | 1.12 ± 0.06 |
| 30% PEG/water | 0.96 ± 0.02 | 1.57 ± 0.05 |
| 40% PEG/water | 1.35 ± 0.04 | 2.25 ± 0.08 |
| 1mM Gd-DTPA/water | 4.68 ± 0.18 | 5.65 ± 0.03 |
| 10% PEG/water/1mM Gd-DTPA | 5.58 ± 0.23 | 6.86 ± 0.02 |
| 20% PEG/water/1mM Gd-DTPA | 7.19 ± 0.53 | 8.69 ± 0.07 |
| 30% PEG/water/1mM Gd-DTPA | 9.42 ± 0.58 | 12.11 ± 0.08 |
| 40% PEG/water/1mM Gd-DTPA | 12.81 ± 0.72 | 17.62 ± 0.15 |

### Example 2

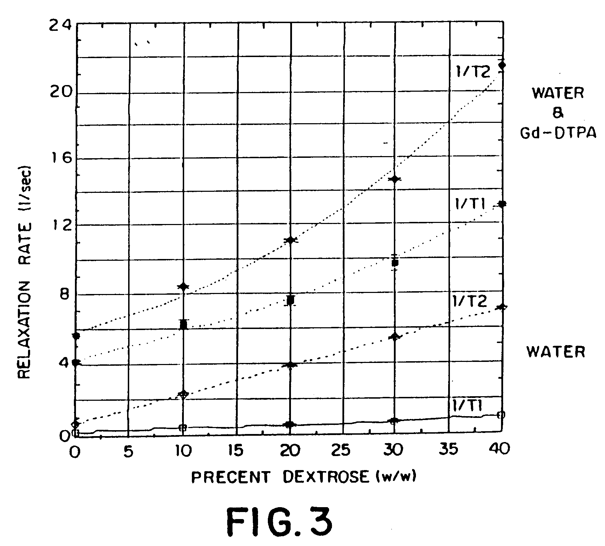

Example 1 was substantially repeated, except that the polymer dextrose was employed instead of PEG. The results are shown in Figure 3. As the results indicate, in the presence of dextrose the relaxivity of both water and Gd-DTPA is increased.

As Figure 3 illustrates, again, as with PEG, the relaxivity of the dextrose and Gd-DTPA solution is larger than the sum of the individual relaxation rates.

### Example 3

Example 1 was substantially repeated except that an aqueous solution of 1mM Gd-DTPA, 30% (w/w) PEG 8000 and 10% (w/w) dextrose was prepared and T1 was reported. A T1 relaxation rate of 11.67 ± 1.09 (1/sec) at 0.5 T was observed.

Examples 1, 2 and 3 show that different polymers or may be used to preferentially alter the T1 or T2 relaxation rates of a solution. For example, as shown in Example 3, a solution of 1mM Gd-DTPA and 30% PEG 8000 and 10% dextrose exhibits a T1 relaxation rate of 11.67 ± 1.09 (1/sec) at 0.5 T. By comparison, 1mM Gd-DTPA and 30% PEG 8000 in solution exhibits a T1 relaxation rate of 9.42 ± 0.58 (1/sec) at 0.5 T, and 1mM Gd-DTPA and 10% dextrose in solution exhibits a T1 relaxation rate of 2.33 ± 0.02 (1/sec) at 0.5 T.

### Example 4

Example 1 was substantially repeated, except that 10% (w/w) cellulose, a low toxicity polymer that is not degraded within the gastrointestinal tract, was employed, in aqueous solution with and without different concentrations of a ferrite contrast agent and with and without carbon dioxide gas.

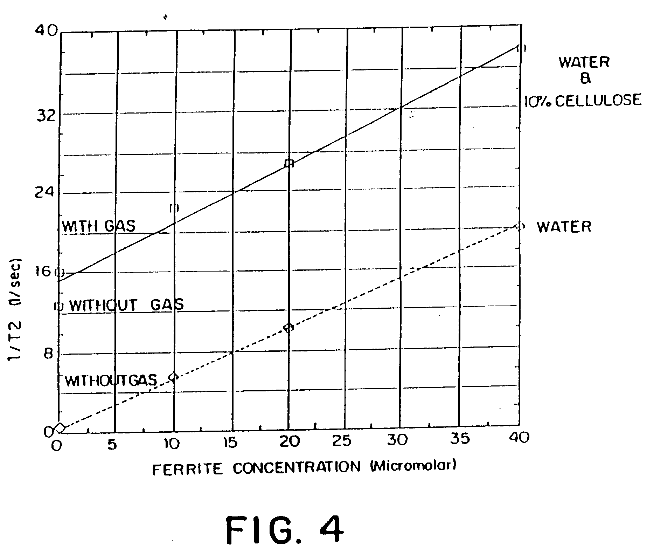

The results are shown in Table 2 and Figure 4. As the results indicate, cellulose is also an efficient T2 relaxation agent, and the T2 relaxivity of cellulose may be improved by mixing with a gas such as carbon dioxide. In addition, the results show that cellulose may be combined with a superparamagnetic contrast agent such as ferrites such that the combined polymer/ferrite contrast agent, whether mixed with gas or not, is superior in terms of relaxivity, as compared with either the polymer or ferrite alone. Specifically, from the results, it can be seen that the T2 relaxivity for a sample containing 10% cellulose with 10mM ferrites after gassification has a higher T2 relaxivity than a 40mM dispersion of ferrites in water. The immediate conclusion is that it would be possible to reduce the dose of ferrites administered by at least a factor of 4 and still obtain the same degree of contrast enhancement. This would have clear benefits in terms of decreasing the potential toxicity of the contrast agent.

**Table 2**

| Relaxivities at 0.5T for water/ferrite and cellulose/water/ferrite mixtures | | |
|---|---|---|
| Sample | 1/T1(1/sec) | 1/T2(1/sec) |
| water | 0.27 ± 0.05 | 0.55 ± 0.06 |
| + 10mM ferrites | 1.16 ± 0.01 | 5.45 ± 0.07 |
| + 20mM ferrites | 1.92 ± 0.03 | 10.20 ± 0.10 |
| + 40mM ferrites | 3.60 + 0.10 | 20.24 ± 0.43 |
| 10 % cellulose in water | - | 12.76 ± 0.08 |
| + pressurized with gas | 0.76 ± 0.01 | 15.95 ± 0.05 |
| gas + 10mM ferrites | 1.98 ± 0.03 | 22.82 ± 0.62 |
| gas + 20mM ferrites | 2.41 ± 0.03 | 26.75 ± 0.32 |
| gas + 40mM ferrites | 4.15 ± 0.11 | 37.42 ± 0.94 |

## Claims

1. A contrast medium for magnetic resonance imaging comprising an aqueous solution or suspension of a biocompatible non-cross-linked polymer and a biocompatible gas.

2. A contrast medium of Claim 1 wherein the polymer is synthetic.

3. A contrast medium according to Claim 1 or Claim 2 wherein the biocompatible gas is selected from air,oxygen, carbon dioxide, nitrogen, xenon, neon and argon.

4. A contrast medium of any of Claims 1 to 3 wherein the polymer is selected from polyethylenes, polyoxyethylenes, polypropylenes, pluronic acids, pluronic alcohols, polyvinyls and polyvinylpyrrolidones.

5. A contrast medium of Claim 4 wherein the polymer is polyethylene glycol.

6. A contrast medium of any of Claims 1 to 5 wherein the contrast medium further comprises an MRI contrast agent in admixture with the polymer

7. A contrast medium of any preceding Claim wherein the contrast agent is selected from superparamagnetic, superparamagnetic, and proton density contrast agents.

8. A contrast medium of Claim 7 wherein the MRI contrast agent is the paramagnetic agent Mn (II).

9. A contrast medium of any of Claims 1 to 8 wherein the polymer is selected from arabinans, fructans, fucans, galactans, glacturonans, glucans, mannans, xylans, levan, fucoidan, carrageenan, galactocarolose, pectic acid, amylose, pullulan, glycogen, amylopectin, cellulose, carboxylmethylcellulose, hydroxypropyl methylcellulose, dextran, pustulan, chitin, agarose, keratan, chondroitin, dermatan, hyaluronic acid, alginic acid, and other homopolymers or heteropolymers containing at least one or more aldose, ketose, acid or amine selected from erythrose, threose, ribose, arabinose, xylose, lyxose, allose, altrose, glucose, mannose, gucose, idose, galactose, talose, erythrulose, ribulose, xylulose, psicose, fructose, sorbose, tagatose, glucuronic acids, gluconic acid, glucaric acid, galacturonic acid, mannuronic acid, glucosamine, galactosamine and neuraminic acid.

10. A contrast medium of Claim 9 wherein the polymer is selected from polygalacturonic acid and dextran.

11. A contrast medium of any preceding Claim wherein the polymer has a relatively high water binding capacity and a limited ability for ion complexation.

12. Use of a magnetic resonance imaging medium as defined in any one of claims 1 to 11 for the manufacture of a diagnostic contrast agent for use in a method of *in vivo* nuclear magnetic resonance imaging of a subject.

## Patentansprüche

1. Kontrastmittel für die magnetische Resonanz-Abbildung (das magnetische Resonanz-Imaging, MRI), wobei das Kontrastmittel eine wäßrige Lösung oder Suspension eines biokompatiblen, nicht-vernetzten Polymers und ein biokompatibles Gas umfaßt.

2. Das Kontrastmittel nach Anspruch 1, wobei das Polymer synthetisch ist.

3. Das Kontrastmittel nach Anspruch 1 oder 2, wobei das biokompatible Gas ausgewählt ist aus Luft, Sauerstoff, Kohlendioxid, Stickstoff, Xenon, Neon und Argon.

4. Das Kontrastmittel nach einem der Ansprüche 1 bis 3, wobei das Polymer ausgewählt ist aus Polyethylenen, Polyoxyethylenen, Polypropylenen, Pluronsäuren, Pluronalkoholen, Polyvinylen und Polyvinylpyrrolidonen.

5. Das Kontrastmittel nach Anspruch 4, wobei das Polymer Polyethylenglykol ist.

6. Das Kontrastmittel nach einem der Ansprüche 1 bis 5, wo-bei das Kontrastmittel außerdem ein MRI Kontrastagens in Abmischung mit dem Polymer umfaßt.

7. Das Kontrastmittel nach einem der vorhergehenden Ansprüche, wobei das Kontrastagens ausgewählt ist aus paramagnetischen, superparamagnetischen und Protonen-Dichte-Kontrastagentien.

8. Das Kontrastmittel nach Anspruch 7, wobei das MRI Kontrastagens das paramagnetische Agens Mn(II) ist.

9. Das Kontrastmittel nach einem der Ansprüche 1 bis 8, wo-bei das Polymer ausgewählt ist aus Arabinanen, Fructanen, Fucanen, Galaktanen, Galacturonanen, Glucanen, Mannanen, Xylanen, Lävan, Fucoidan, Karraghen, Galaktocarolose, Pektinsäure, Amylose, Pullulan, Glykogen, Amylopektin, Cellulose, Carboxymethylcellulose, Hydroxypropylmethylcellulose, Dextran, Pustulan, Chitin, Agarose, Keratan, Chondroitin, Dermatan, Hyaluronsäure, Alginsäure und an-dere Homopolymere und Heteropolymere, die mindestens eine Aldose-, Ketose-, Säure- oder Amin-Funktion enthalten, ausgewählt aus Erythrose, Threose, Ribose, Arabinose, Xy-lose, Lyxose, Allose, Altrose, Glukose, Mannose, Gucose, Idose, Galaktose, Talose, Erythrulose, Ribulose, Xylulose, Psicose, Fruktose, Sorbose, Tagatose, Glukuronsäure, Glukonsäure, Glukarsäure, Galakturonsäure, Mannuronsäure, Glukosamin, Galaktosamin und Neuraminsäure.

10. Das Kontrastmittel nach Anspruch 9, wobei das Polymer ausgewählt ist aus Polygalakturonsäure und Dextran.

11. Das Kontrastmittel nach einem der vorhergehenden Ansprüche, wobei das Polymer eine relativ hohe Wasserbindungskapazität und eine begrenzte Fähigkeit zur Ionenkomplexierung hat.

12. Verwendung des magnetischen Resonanzabbildungsmittels, wie es in einem der Ansprüche 1 bis 11 definiert ist, für die Herstellung eines diagnostischen Kontrastagens zur Verwendung in einem Verfahren des in vivo kernmagnetischen Resonanz-Abbildens eines Lebewesens.

## Revendications

1. Milieu de contraste pour imagerie par résonance magnétique, comprenant une solution ou suspension aqueuse d'un polymère non réticulé biocompatible et un gaz biocompatible.

2. Milieu de contraste selon la revendication 1, dans lequel le polymère est synthétique.

3. Milieu de contraste selon l'une des revendications 1 et 2, dans lequel le gaz biocompatible est choisi parmi l'air, l'oxygène, le dioxyde de carbone, l'azote, le xénon, le néon et l'argon.

4. Milieu de contraste selon l'une quelconque des revendications 1 à 3, dans lequel le polymère est choisi parmi les polyéthylènes, les polyoxyéthylènes, les polypropylènes, les acides pluroniques, les alcools pluroniques, les polyvinyles et les polyvinylpyrrolidones.

5. Milieu de contraste selon la revendication 4, dans lequel le polymère est le polyéthylèneglycol.

6. Milieu de contraste selon l'une quelconque des revendications 1 à 5, dans lequel le milieu de contraste comprend également un agent de contraste pour imagerie par résonance magnétique (MRI) en mélange avec le polymère.

7. Milieu de contraste selon l'une quelconque des revendications précédentes, dans lequel l'agent de contraste est choisi parmi les agents de contraste paramagnétiques, supraparamagnétiques et modifiant la densité protonique.

8. Milieu de contraste selon la revendication 7, dans lequel l'agent de contraste pour MRI est l'agent paramagnétique Mn(II).

9. Milieu de contraste selon l'une quelconque des revendications 1 à 8, dans lequel le polymère est choisi parmi les arabinanes, les fructanes, les fucanes, les galactanes, les galacturonanes, les glucanes, les mannanes, les xylanes, le levane, le fucoïdane, la carraghénine, le galactocarolose, l'acide pectique, l'amylose, le pullulane, le glycogène, l'amylopectine, la cellulose, la carboxyl méthylcellulose, l'hydroxypropyl méthylcellulose, le dextrane, le pustulane, la chitine, l'agarose, le kératane, la chondroïtine, le dermatane, l'acide hyaluronique, l'acide alginique, et autres homopolymères ou hétéropolymères contenant au moins un ou plusieurs aldoses, cétoses, acides ou amines choisis parmi l'érythrose, le thréose, le ribose, l'arabinose, le xylose, le lyxose, l'allose, l'altrose, le glucose, le mannose, le gulose, l'idose, le galactose, le talose, l'érythrulose, le ribulose, le xylulose, le psicose, le fructose, le sorbose, le tagatose, les acides glucuroniques, l'acide gluconique, l'acide glucarique, l'acide galacturonique, l'acide mannuronique, la glucosamine, la galactosamine et l'acide neuraminique.

10. Milieu de contraste selon la revendication 9, dans lequel le polymère est choisi parmi l'acide polygalacturonique et le dextrane.

11. Milieu de contraste selon l'une quelconque des revendications précédentes, dans lequel le polymère a une capacité de liaison avec l'eau relativement élevée et une capacité limitée à complexer les ions.

12. Utilisation d'un milieu pour imagerie par résonance magnétique tel que défini à l'une quelconque des revendications 1 à 11, pour la fabrication d'un agent de contraste pour diagnostic, destiné à être utilisé dans un procédé d'imagerie par résonance magnétique nucléaire *in vivo* d'un sujet.
